# EUROPEAN PATENT APPLICATION

(11) **EP 3 583 955 A1**
(43) Date of publication of application: **25.12.2019**
(21) Application number: 18754947.2
(22) Date of filing: 02.02.2018
(51) Int. Cl.: A61K 47/69, A61K 47/10

(54) **EXOSOME-BASED NANOPARTICLE COMPOSITE AND METHOD FOR PREPARING SAME**

(30) Priority: 15.02.2017 KR 20170020293
(71) Applicant: Korea University Research and Business Foundation, Sejong Campus, Sejong-si 30019 (KR)
(72) Inventor: YUK, Soon Hong, Yongin-si Gyeonggi-do 16874 (KR)
(74) Representative: Grosse, Felix Christopher
(86) International application number: PCT/KR2018/001432
(87) International publication number: WO 2018/151445

(57) **Abstract**

The present invention relates to an exosome-based nanoparticle composite and a method for preparing the same. More specifically, the present invention relates to an exosome-based nanoparticle composite that uses exosomes isolated from cells and a biocompatible polymer to achieve improved *in vivo* stability and redispersibility, and a method for preparing the nanoparticle composite. The nanoparticle composite of the present invention exhibits a stable and continuous drug release pattern irrespective of the solubility of the drug and has excellent tumor targeting due to its good *in vivo* stability and improved redispersibility in aqueous solution.

## Description

### Technical Field

The present invention relates to an exosome-based nanoparticle composite and a method for preparing the same. More specifically, the present invention relates to an exosome-based nanoparticle composite that uses exosomes isolated from cells and a biocompatible polymer to achieve improved *in vivo* stability and redispersibility, and a method for preparing the nanoparticle composite.

### Background Art

Drugs are broadly classified into two groups: hydrophobic and hydrophilic. Hydrophobic drugs are not effective for *in vivo* delivery because of their limited solubility in aqueous solution. Hydrophilic drugs exert their pharmacological effects at the initial stage of administration due to their high solubility but need to be frequently administered because of difficulties encountered in exerting their pharmacological effects for a sustained period of time. To overcome these difficulties, attempts have been made to develop various drug delivery systems using nanoparticles and many studies have been conducted on the use of polymer drug delivery systems, lipid drug delivery systems, and nanotubes. The polymer drug delivery systems are exemplified by polymer-drug conjugates, polymeric micelles, and dendrimers.

However, the drug delivery systems show fast drug release profiles due to their instability in aqueous solution. Particularly, when a water-soluble drug or protein drug and antibodies are filled in the drug delivery systems, the high solubility of the drug delivery systems in aqueous solution causes a rapid release of the drug, making it difficult to achieve the desired pharmacological effects of the drug. One approach to overcome this difficulty is to repeatedly administer the drug. High costs of the protein drug or antibodies are expected to lead to an increase in medical expenses.

Liposomes have received attention as effective drug delivery systems because they consist of several types of lipid molecules and phospholipids constituting liposomes are almost non-toxic or harmless to humans. Numerous studies have been conducted aimed at ensuring physical stability of liposome-based particles, for example, by adding a special surfactant to induce an increase in the stability of liposomes. The addition of electrostatically charged lipids to and the introduction of a sterol, anionic lipids or sphingolipids into liposomal constituents have been proposed as alternative approaches to improve the physical stability of liposome-based particles. Although studies have been continued to improve the stability of liposomes, their utilization in the pharmaceutical field is limited because none of them have been able to provide sufficient physical stability of liposomes.

In an effort to improve the physical stability of liposomes, the present inventors have succeeded in developing core/shell structured drug delivery systems consisting of lecithin cores constituting liposomes and Poloxamer shells, as disclosed in Korean Patent No. 10-0792557. However, the drug delivery systems are inevitably limited in terms of biocompatibility and bioavailability because they are not based on human-derived cells but on liposomes.

On the other hand, a tumor targeting function is imparted to injectable anticancer drug delivery systems in order to minimize the side effects of the anticancer drugs. In recent years, however, the limitations of the enhanced permeation and retention (EPR) effect, a major delivery mechanism of passive tumor targeting playing a part in the establishment of tumor targeting, have been exposed.

Under such circumstances, there is an urgent need to develop a new type of biocompatible particles that can overcome limitations in terms of biocompatibility and bioavailability to achieve both good *in vivo* stability and high redispersibility in aqueous solution and can achieve active tumor targeting without complicated surface modification.

### (Patent Document 1) Korean Patent No. 10-0792557

### Detailed Description of the Invention

### Problems to be Solved by the Invention

The present invention has been made in an effort to solve the above-described problems and intends to provide a nanoparticle composite that is highly stable *in vivo* and can be redispersed in aqueous solution to exhibit a stable and continuous drug dissolution pattern and has excellent tumor targeting.

The present invention also intends to provide a method for preparing the nanoparticle composite.

### Means for Solving the Problems

One aspect of the present invention provides a core/shell nanoparticle composite including cell line-derived, drug-loaded exosome cores and PEO-PPO-PEO copolymer shells associated on the surface of the cell line-derived exosome cores.

A further aspect of the present invention provides a method for preparing a core/shell nanoparticle composite, including (a) isolating exosomes from a cell line by centrifugation, (b) mixing the isolated exosomes with a drug to load the drug into the exosomes, and (c) mixing the drug-loaded exosomes with an aqueous solution of a PEO-PPO-PEO copolymer, followed by freeze-drying.

### Effects of the Invention

The use of the exosomes derived from human cells and the biocompatible polymer makes the nanoparticle composite of the present invention highly stable *in vivo* and redispersible in aqueous solution so that the nanoparticle composite exhibits a stable and continuous drug dissolution pattern irrespective of the solubility of the drug and has excellent tumor targeting.

### Brief Description of the Drawings

Fig. 1 is a schematic diagram showing a core/shell nanoparticle composite according to the present invention.
Fig. 2 is a schematic diagram showing a method for preparing a core/shell nanoparticle composite according to the present invention.

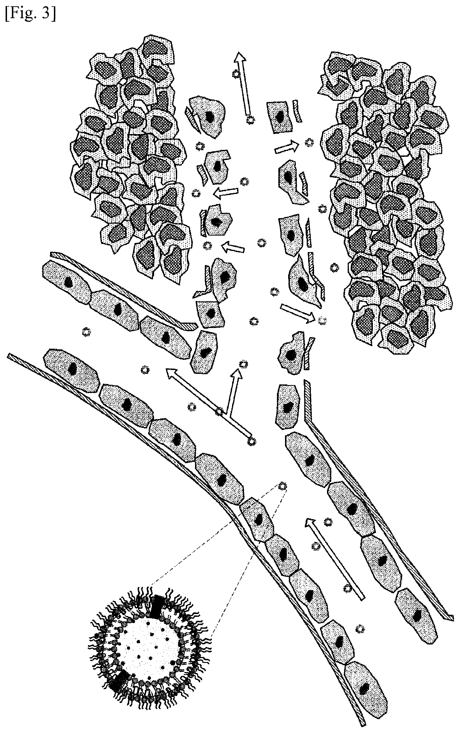

Fig. 3 is a schematic diagram showing the *in vivo* behavior of a conventional core/shell nanoparticle composite based on the EPR effect.
Fig. 4 is a TEM image of a core/shell nanoparticle composite prepared in Example 1-1.
Fig. 5 is a TEM image of a core/shell nanoparticle composite prepared in Example 1-2.
Fig. 6 shows the particle size distribution of a core/shell nanoparticle composite prepared in Example 1-1.
Fig. 7 shows the time-dependent drug release profiles of core/shell nanoparticle composites prepared in Example 1-1 and Comparative Example 1.
Fig. 8 shows the biodistributions of core/shell nanoparticle composites prepared in Example 2 and Comparative Example 2 in tumor-bearing animal models.

### Best Mode for Carrying out the Invention

The present invention will now be described in more detail.

Conventional liposome-based drug delivery systems use liposomes composed of lecithin extracted from soybean or egg yolk. However, the drug delivery systems are inevitably limited in terms of biocompatibility and bioavailability because they are not based on human-derived cells but on liposomes.

Thus, there is an urgent need to develop a new type of biocompatible particles that can overcome limitations in terms of biocompatibility and bioavailability to achieve both good *in vivo* stability and high redispersibility in aqueous solution and can achieve the EPR effect and active tumor targeting without complicated surface modification.

Under such technical background, the present inventors have conducted research on a new type of biocompatible particles, and as a result, found that a drug delivery system based on cell line-derived exosomes is highly stable *in vivo* and can be redispersed in aqueous solution and exhibits a continuous drug dissolution pattern and markedly improved tumor targeting compared to liposome-based drug delivery systems. The present invention has been accomplished based on this finding.

The present invention provides a core/shell nanoparticle composite including cell line-derived, drug-loaded exosome cores and PEO-PPO-PEO copolymer shells associated on the surface of the cell line-derived exosome cores.

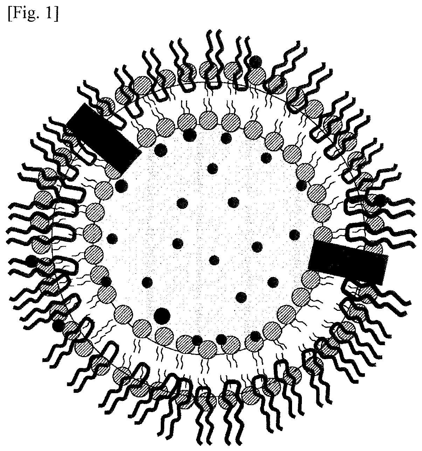

Fig. 1 is a schematic diagram showing a core/shell nanoparticle composite according to the present invention in which a PEO-PPO-PEO copolymer is associated on the surface of an exosome core.

The term "association" as used herein refers to a state in which the PEO-PPO-PEO copolymer is bound to the surface of the exosome cores by intermolecular interaction or is entangled (or intervened) with at least some portions of the phospholipid bilayer of the exosome cores.

Referring to FIG. 1, the PEO-PPO-PEO copolymer is intervened with the surface of the drug-loaded exosome core and at least some portions of the phospholipid bilayer of the exosome to stabilize the surface of the exosome. The PEO-PPO-PEO copolymer penetrates and is strongly intervened with the bilayer of the exosome. The PEO-PPO-PEO copolymer forms a surface layer on the outer wall of the exosome to protect the structure of the exosome from external factors that may make the structure of the exosome unstable. That is, the PEO-PPO-PEO copolymer serves to stably maintain the structure of the exosome.

In addition, the core/shell nanoparticle composite of the present invention is highly dispersible without agglomeration. Therefore, the core/shell nanoparticle composite can be readily redispersed when dissolved in a solution such as an aqueous solution for *in vivo* administration.

The core/shell nanoparticle composite of the present invention is in the form of a fine powder with a particle size of about 100 to about 1,000 nm, preferably about 300 to about 700 nm. In addition, the core/shell nanoparticle composite of the present invention has good storage stability because the drug is protected by the exosome cores and the PEO-PPO-PEO copolymer shells.

The core/shell nanoparticle composite of the present invention can be stored in a dry state for a long time and is thus convenient to store, transport, and administer.

The drug loaded into the exosome cores is not limited to a particular type and examples thereof include poorly soluble drugs, water-soluble drugs, ionic drugs, protein drugs, antibodies, and contrast agents. The poorly soluble drugs refer to drugs that are difficult to solubilize due to their low solubility in water. Examples of the poorly soluble drugs include, but are not limited, anticancer agents and therapeutic agents for cardiovascular diseases such as arteriosclerosis and hyperlipidemia. Specific examples of the poorly soluble drugs include, but are not limited, paclitaxel, docetaxel, pamoxin, anastrozole, carboplatin, topotecan, belotecan, imatinib, irinotecan, floxuridine, vinorelbine, gemcitabine, leuprolide, flutamide, zoledronate, methotrexate, camptothecin, cisplatin, vincristine, hydroxyurea, streptozocin, valrubicin, lovastatin, simvastatin, fluvastatin, atorvastatin, pitavastatin, pravastatin, and rosuvastatin.

Examples of the ionic drugs include, but are not limited to, doxorubicin-HCl, doxazosin HCl, methylphenidate, oxybutynin chloride, pseudoephedrine HCl, albuterol sulfate, and bupivacaine-HCl.

Examples of the proteins and the antibodies include, but are not limited to, insulin, exenatide, human growth hormone (Hgh), vascular endothelial growth factor (VEGF), epidermal growth factor (EGF), and tumor necrosis factor-a (TNF-α).

For example, the contrast agents may include one or more paramagnetic materials selected from the group consisting of manganese, gadolinium, erbium, chromium, iron, cobalt, nickel, the elements of the lanthanide series, the elements of the actinide series, and combinations thereof. An exemplary contrast agent is 1,4,7,10-tetraazacyclododecane-1,4,7,10-tetraacetic acid gadolinium (III) (Gd-DOTA) complex. The Gd-DOTA complex is a magnetic resonance imaging (MRI) contrast agent that is effective in enhancing molecular imaging, particularly imaging of tumor-bearing sites when filled in the exosome cores of the core/shell nanoparticle composite according to the present invention.

The PEO-PPO-PEO copolymer may be a triblock copolymer represented by Formula 1:

[Formula 1] **HO-(C₂H₄O)ₐ(C₃H₆O)_{b}(C₂H₄O)_{c}-H**

wherein **b** is an integer of 10 or more and **a**+**c** is a number such that the end blocks **(C₂H₄O)ₐ** and **(C₂H₄O)_{c}** account for 5 to 95% by weight, preferably 20 to 90% by weight of the copolymer.

The properties of the PEO-PPO-PEO copolymer depend on the ratio of the polyoxypropylene blocks to the polyoxyethylene blocks, that is, the ratio of **b** to **a**+**c** in Formula 1. The PEO-PPO-PEO copolymer may be prepared by a known method described in the literature or may be a commercially available product. The molecular weight of the PEO-PPO-PEO copolymer is not particularly limited and may be, for example, from about 1,000 to about 16,000.

Meanwhile, the PEO-PPO-PEO copolymer is a commercial polymer known as Poloxamer or Pluronic. Poloxamers are solids at room temperature and tend to be soluble in water and ethanol. According to one embodiment of the present invention, the PEO-PPO-PEO copolymer may be Poloxamer 68, Poloxamer 127, Poloxamer 188, Poloxamer 237, Poloxamer 338 or Poloxamer 407 but is not limited thereto. For example, Poloxamer 188 is the polymer of Formula 1 wherein **b** is 30 and **a**+**c** is 75. The molecular weight of Poloxamer 188 is approximately 8,350.

The use of the cell line-derived exosomes in the core/shell nanoparticle composite of the present invention contributes to a considerable improvement in biocompatibility. Exosomes that can be derived from cell lines may be used in the present invention. The cell lines are preferably macrophage or cancer cell lines.

RAW264.7 macrophage cell line may be used in the present invention. When exosomes derived from RAW264.7 macrophage cell line are used, the core/shell nanoparticle composite of the present invention can exhibit a markedly improved release behavior compared to liposome-based composites.

All cancer cell lines may be used in the present invention. The cancer cell lines are preferably breast cancer cell lines or squamous cell carcinoma cell lines. MDA-MB231 breast cancer cell line and SCC-7 squamous cell carcinoma cell line may be used in the present invention. The core/shell nanoparticle composite of the present invention may be used for an anticancer drug delivery system or a pharmaceutical composition for cancer treatment. In this case, the exosomes are preferably isolated from a cell line of the same type of cancer being treated in order to achieve significantly improved tumor targeting. A core/shell nanoparticle composite based on exosomes derived from breast cell line MDA-MB231 has better tumor targeting to breast cancer than a core/shell nanoparticle composite based on exosomes derived from SCC-7 cell line, which is confirmed in the Examples section that follows.

For *in vivo* administration, the core/shell nanoparticle composite of the present invention can be dispersed in water or any other solvent. Since the drug loaded into the exosome cores exists in a stabile state, its bioavailability can be significantly improved regardless of its inherent solubility. The use of the cell line-derived exosomes can also lead to a significant improvement in biocompatibility. Since the core/shell nanoparticle composite of the present invention exhibits a sustained release dissolution pattern compared to liposome-based drug delivery systems, which is evident from the results in the following Examples section, it can be usefully applied to sustained release preparations where long-term *in vivo* sustained release is needed. For example, the core/shell nanoparticle composite of the present invention can sustainably release the drug *in vivo* at a constant rate for several days to several months after *in vivo* administration.

The core/shell nanoparticle composite of the present invention can achieve the EPR effect and active tumor targeting without complicated surface modification, which is demonstrated by the following Examples section where the core/shell nanoparticle composite of the present invention has improved tumor targeting compared to liposome-based drug delivery systems. Therefore, the core/shell nanoparticle composite of the present invention can be used as an effective drug delivery system.

The core/shell nanoparticle composite of the present invention can be formulated into preparations such as granules, capsules or tablets for oral administration or preparations for parenteral administration such as intravenous, subcutaneous or intramuscular injection according to suitable techniques known in the art. The core/shell nanoparticle composite of the present invention can be designed as an effective drug delivery system with various pharmaceutically acceptable excipients according to a suitable formulation technique known in the pharmaceutical field.

The core/shell nanoparticle composite of the present invention is in the form of a highly flowable powder, which is advantageous for long-term storage. Therefore, a pharmaceutical preparation including the core/shell nanoparticle composite of the present invention has improved storage stability and is at very low risk of spoilage even during long-term transport and storage.

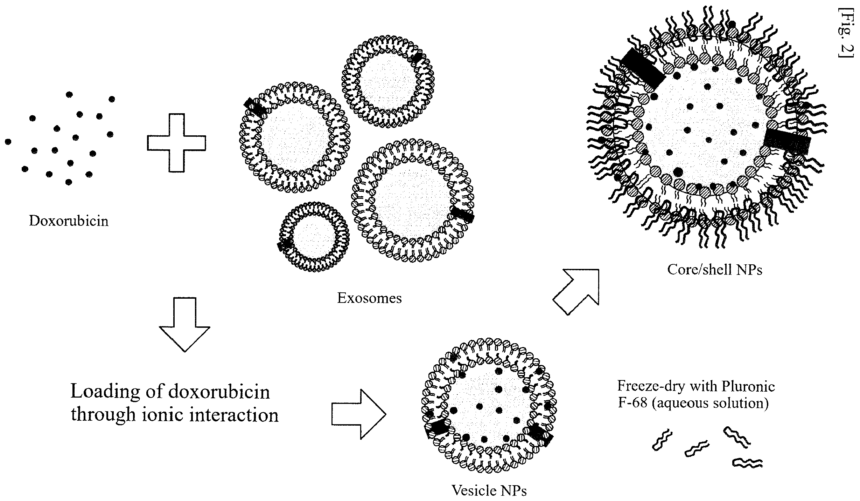

The present invention also provides a method for preparing a core/shell nanoparticle composite, including (a) isolating exosomes from a cell line by centrifugation, (b) mixing the isolated exosomes with a drug to load the drug into the exosomes, and (c) mixing the drug-loaded exosomes with an aqueous solution of a PEO-PPO-PEO copolymer, followed by freeze-drying. (Fig. 2).

First, in step (a), exosomes are isolated from a cell line by centrifugation. The centrifugation can be performed by a suitable method known in the art, for example, differential ultracentrifugation (C. Thery, S. Amigorena, G. Raposo, A. Clayton, Isolation and characterization of exosomes from cell culture supernatants and biological fluids, Current protocols in cell biology, Chapter 3 (2006) Unit 3 22.).

Next, in step (b), the isolated exosomes are mixed with a drug to load the drug into the exosomes. The drug is not limited to a particular type and examples thereof include poorly soluble drugs, water-soluble drugs, ionic drugs, protein drugs, antibodies, and contrast agents.

Then, in step (c), the drug-loaded exosomes are mixed with an aqueous solution of a PEO-PPO-PEO copolymer with stirring, followed by freeze-drying. The drug-loaded exosomes form cores and the PEO-PPO-PEO copolymer is associated on the surface of the exosome cores to form shells. The resulting core/shell nanoparticle composite is powdered by freeze-drying.

Here, the aqueous PEO-PPO-PEO copolymer solution may include 1 to 30% by weight of the PEO-PPO-PEO copolymer.

In step (b), the drug-loaded exosomes and the PEO-PPO-PEO copolymer may be mixed in a weight ratio of 1:0.1 to 1:99, preferably 1:1 to 1:20, more preferably 1:1 to 1:5. If the amount of the PEO-PPO-PEO copolymer is too small, the effect of the PEO-PPO-PEO copolymer on the stabilization of the surface of the exosomes is negligible. Meanwhile, if the amount of the PEO-PPO-PEO copolymer is too large, the release of the filled drug is suppressed, with the result that the desired therapeutic effect of the drug cannot be achieved.

The PEO-PPO-PEO copolymer is associated on the surface and at least some portions of the phospholipid bilayer of the drug-loaded exosomes to maintain the structure of the exosomes.

The core/shell nanoparticle composite prepared by the method of the present invention is in the form of a fine powder that is highly dispersible without agglomeration. Thus, the core/shell nanoparticle composite can be readily redispersed when dissolved in a solution such as an aqueous solution for *in vivo* administration. In addition, the method of the present invention avoids the need to use an organic solvent or other excipients that may have a harmful influence on humans and enables the preparation of the core/shell nanoparticle composite in a simple manner using the cell line-derived exosomes and the biocompatible PEO-PPO-PEO copolymer, achieving both *in vivo* stability and ease of processing. Furthermore, the core/shell nanoparticle composite can be used in a variety of pharmaceutical preparations due to its improved storage stability.

### Mode for Carrying out the Invention

The present invention will be explained in more detail with reference to the following examples. It will be evident to those skilled in the art that these examples are merely for illustrative purposes and are not intended to limit the scope of the present invention.

### <EXAMPLES>

### Example 1: Preparation of core/shell nanoparticle composite based on exosomes derived from RAW264.7 cell line

### (1) Example 1-1

First, 10 mg of exosomes were isolated from RAW264.7 cell line by centrifugation. The isolated exosomes were mixed with 10 mg of doxorubicin-HCl for 2 h to prepare a homogeneous mix. To the mix was added 50 mg of an aqueous solution of 10 wt% Pluronic F-68. The mixture was stirred until complete dissolution. The resulting solution was freeze-dried for 48 h to obtain a core/shell nanoparticle composite in the form of a powder.

### (2) Example 1-2

A core/shell nanoparticle composite in the form of a powder was prepared in the same manner as in Example 1-1, except that an aqueous solution of 10 wt% Pluronic F-127 was used instead of the aqueous solution of 10 wt% Pluronic F-68.

### Example 2: Preparation of core/shell nanoparticle composite based on exosomes derived from MDA-MB231 cell line

First, 10 mg of exosomes were isolated from MDA-MB231 cell line by centrifugation. The isolated exosomes were mixed with 1 mg of Cy 5.5 as a fluorescent dye for biodistribution measurement for 2 h to prepare a homogeneous mix. To the mix was added 50 mg of an aqueous solution of 10 wt% Pluronic F-68. The mixture was stirred until complete dissolution. The resulting solution was freeze-dried for 48 h to obtain a core/shell nanoparticle composite in the form of a powder.

### Comparative Example 1: Preparation of liposome-based core/shell nanoparticle composite

10 mg of doxorubicin-HCl was mixed with liposomes composed of phosphatidylcholine extracted from egg yolk for 2 h to prepare a homogeneous mix. To the mix was added 50 mg of an aqueous solution of 10 wt% Pluronic F-68. The mixture was stirred until complete dissolution. The resulting solution was freeze-dried for 48 h to obtain a core/shell nanoparticle composite in the form of a powder.

### Comparative Example 2: Preparation of core/shell nanoparticle composite based on exosomes derived from SCC-7 cell line

First, 10 mg of exosomes were isolated from SCC-7 cell line by centrifugation. The isolated exosomes were mixed with 1 mg of Cy 5.5 as a fluorescent dye for biodistribution measurement for 2 h to prepare a homogeneous mix. To the mix was added 50 mg of an aqueous solution of 10 wt% Pluronic F-68. The mixture was stirred until complete dissolution. The resulting solution was freeze-dried for 48 h to obtain a core/shell nanoparticle composite in the form of a powder.

### <EXPERIMENTAL EXAMPLES>

### Experimental Example 1: Particle size distribution analysis

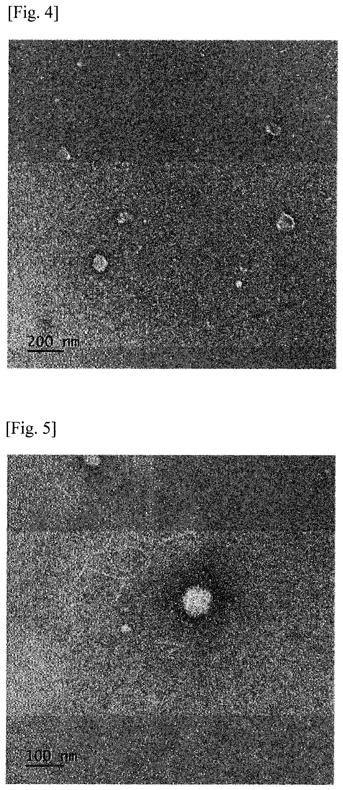

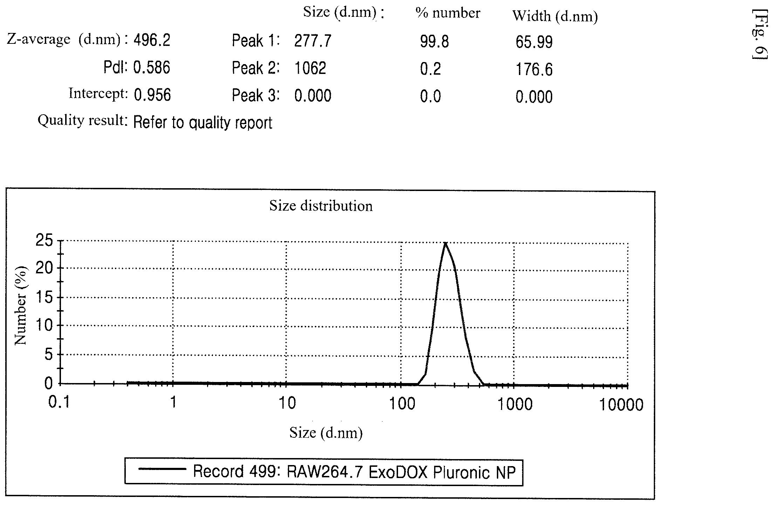

The particle size distribution of the core/shell nanoparticle composite obtained in Example 1-1 is shown in Fig. 6. TEM images of the core/shell nanoparticle composites obtained in Examples 1-1 and 1-2 are shown in Figs. 4 and 5, respectively.

As can be seen from Figs. 4-6, the inventive core/shell nanoparticle composites had particle diameters of ∼500 nm with relatively uniform particle size distributions.

### Experimental Example 2: Drug release pattern analysis

The release pattern of the drug loaded into the inventive core/shell nanoparticle composite was confirmed by the following experiment.

The core/shell nanoparticle composite prepared in Example 1-1 was sampled, placed in a dialysis bag (molecular weight filtration limit: 100,000), and impregnated with 30 ml of phosphate-buffered saline (PBS, pH 7.4). The amount of the sampled nanoparticle composite was determined such that 10 mg of the doxorubicin-HCl was present in the sample. The phosphate-buffered saline was stirred at 100 rpm while maintaining the temperature at 37.5 °C. After the lapse of a predetermined time, 5 ml of the phosphate-buffered saline was sampled. The amount of the drug released was measured by HPLC. After sampling, the remaining phosphate-buffered saline was completely removed and replaced with a fresh one. The amount of the doxorubicin-HCl released was measured using a UV-Vis spectrometer (G1103A, Agilent, USA).

For comparison, each of doxorubicin-HCl (10 mg) and the liposome-based core/shell nanoparticle composite prepared in Comparative Example 1 (containing 10 mg of doxorubicin-HCl) was added to 10 mL of distilled water. The release behavior of the drug in the aqueous solution was measured by the same procedure as described above.

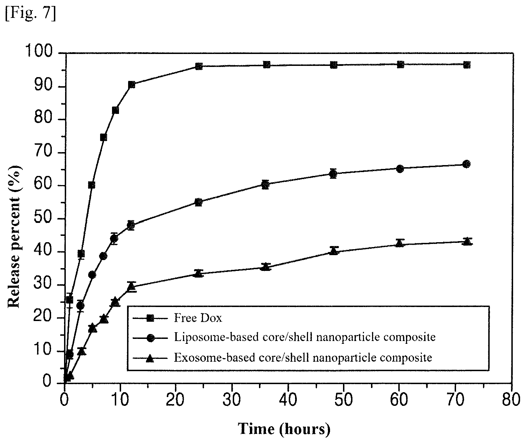

Fig. 7 shows the time-dependent drug release profiles of the core/shell nanoparticle composites prepared in Example 1-1 and Comparative Example 1.

As can be seen from Fig. 7, doxorubicin-HCl was rapidly released at body temperature (37 °C). Specifically, ≥ 95% of the drug was released into phosphate-buffered saline within 24 h. The decreased rate of doxorubicin-HCl was observed with the liposome-based core/shell nanoparticle composite of Comparative Example 1 and 65% of doxorubicin-HCl was released into phosphate-buffered saline within during 72 h.

In contrast, even after 72 h from the start of the release behavior experiment, doxorubicin-HCl was very slowly released and 40% of doxorubicin-HCl was released from the core/shell nanoparticle composite of Example 1, which was based on exosomes derived from RAW264.7 cell line. The release rate of doxorubicin-HCl from the core/shell nanoparticle composite of Example 1 was much lower than that from the liposome-based core/shell nanoparticle composite of Comparative Example 1. The drug was very slowly released at a constant rate from the core/shell nanoparticle composite of Example 1 even after 72 h from the start of the release behavior experiment. These results concluded that the drug release profile of the inventive exosome-based core/shell nanoparticle composite is more stable with sustainability than that of the liposome-based core/shell nanoparticle composite.

### Experimental Example 3: Biodistribution measurement

The *in vivo* behavior of the inventive core/shell nanoparticle composite was investigated through the biodistribution of the inventive core/shell nanoparticle composite by the following experiment.

Specifically, MDA-MB231 cell line was transplanted into C3H/HeN mice, aged 5.5 weeks. When the tumor grew to a size of ∼250-300 mm³, each of the core/shell nanoparticle composite based on exosomes isolated from the MDA-MB231 cell line (Example 2) and the core/shell nanoparticle composite based on exosomes isolated from the SCC-7 cell line (Comparative Example 2) was injected into the mice by tail vein injection. Thereafter, the *in vivo* behavior of the composite was observed using an IVIS SPECTRUM (Xenogen, Alameda, CA) (see Journal of Controlled Release 228 (2016) 141-149).

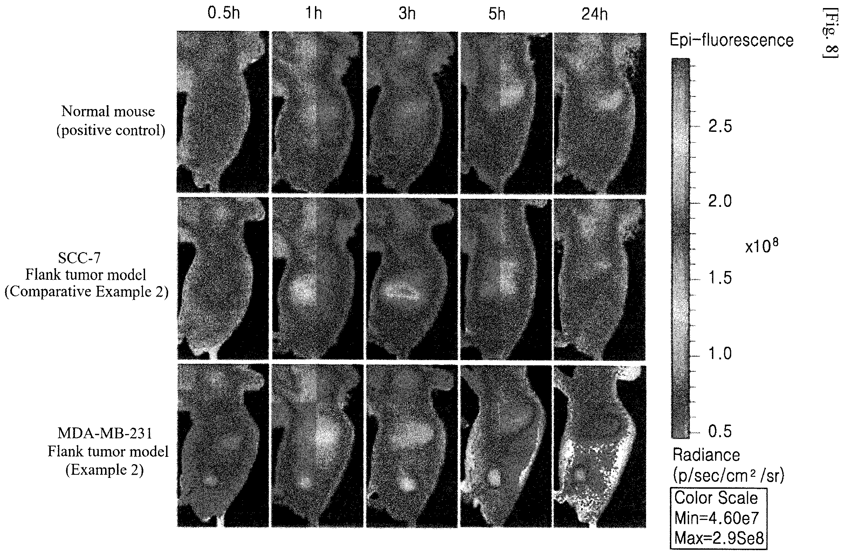

Fig. 8 shows the biodistributions of the core/shell nanoparticle composites prepared in Example 2 and Comparative Example 2 in tumor-bearing animal models.

As confirmed from Fig. 8, the core/shell nanoparticle delivery system based on the exosomes isolated from the breast cancer cell line MDA-MB 231 (Example 2) showed higher tumor targeting than the exosome-based core/shell nanoparticle composite based on the exosomes derived from the SCC-7 cell line (Comparative Example 2), demonstrating that the use of the composite prepared based on the exosomes derived from the same cancer cell line leads to a significant improvement in tumor targeting.

### Industrial Applicability

The nanoparticle composite of the present invention exhibits a stable and continuous drug release pattern irrespective of the solubility of the drug and has excellent tumor targeting due to its good *in vivo* stability and high redispersibility in aqueous solution. Therefore, the nanoparticle composite of the present invention can be widely utilized in the field of drug delivery systems.

## Claims

1. A core/shell nanoparticle composite comprising cell line-derived, drug-loaded exosome cores and PEO-PPO-PEO copolymer shells associated on the surface of the cell line-derived exosome cores.

2. The core/shell nanoparticle composite according to claim 1, wherein the drug is selected from the group consisting of poorly soluble drugs, water-soluble drugs, ionic drugs, protein drugs, antibodies, contrast agents, and mixtures thereof.

3. The core/shell nanoparticle composite according to claim 1, wherein the composite is in the form of a powder.

4. The core/shell nanoparticle composite according to claim 1, wherein the PEO-PPO-PEO copolymer is selected from the group consisting of Poloxamer 68, Poloxamer 127, Poloxamer 188, Poloxamer 237, Poloxamer 338, Poloxamer 407, and mixtures thereof.

5. The core/shell nanoparticle composite according to claim 1, wherein the exosomes are derived from a macrophage or cancer cell line.

6. The core/shell nanoparticle composite according to claim 5, wherein the cancer cell line is a breast cancer cell line or a squamous cell carcinoma cell line.

7. A method for preparing a core/shell nanoparticle composite, comprising (a) isolating exosomes from a cell line by centrifugation, (b) mixing the isolated exosomes with a drug to load the drug into the exosomes, and (c) mixing the drug-loaded exosomes with an aqueous solution of a PEO-PPO-PEO copolymer, followed by freeze-drying.

8. The method according to claim 7, wherein the drug is selected from the group consisting of poorly soluble drugs, water-soluble drugs, ionic drugs, protein drugs, antibodies, contrast agents, and mixtures thereof.

9. The method according to claim 7, wherein the PEO-PPO-PEO copolymer is selected from the group consisting of Poloxamer 68, Poloxamer 127, Poloxamer 188, Poloxamer 237, Poloxamer 338, Poloxamer 407, and mixtures thereof.

10. The method according to claim 7, wherein the aqueous PEO-PPO-PEO copolymer solution comprises 1 to 30% by weight of the PEO-PPO-PEO copolymer.

11. The method according to claim 7, wherein, in step (b), the drug-loaded exosomes and the PEO-PPO-PEO copolymer are mixed in a weight ratio of 1:0.1 to 1:99.
